# EUROPÄISCHE PATENTANMELDUNG

(11) **EP 0 630 625 A2**
(43) Veröffentlichungstag der Anmeldung: **28.12.1994**
(21) Anmeldenummer: 94108301.6
(22) Anmeldetag: 30.05.1994
(51) Int. Cl.: A61F 2/44

(54) **Implantat für den Ersatz von Wirbelkörpern**

(30) Priorität: 24.06.1993 DE 4320987; 19.05.1994 DE 4417629
(71) Anmelder: MAN Ceramics GmbH, 94453 Deggendorf (DE)
(72) Erfinder: Bertagnoli, Rudolf, Dr. med., D-37073 Göttingen (DE)

(57) **Zusammenfassung**

Für den Ersatz von mehreren Wirbelkörpern (25, 26) eines Menschen wird ein leicht gekrümmtes, annähernd zylindrisches Implantat (10) vorgeschlagen, an dessen domartigen Enden (14, 15) je ein Stützelement (17, 18) mit an die Anatomie des menschlichen Rückgrads anpaßbarer Winkellage befestigt wird.

## Beschreibung

Die Erfindung bezieht sich auf ein Implantat für den Ersatz von Wirbelkörpern, bestehend aus einem zentralen Hauptträger, an dessen beiden Enden je ein Stützelement mit einstellbarer Winkellage der Auflageebene für einen anschließenden Wirbelkörper vorgesehen ist.

Wenn in der menschlichen Wirbelsäule einzelne oder mehrere Knochenelemente (Wirbelkörper) durch Tumore oder Gewalteinwirkung ihre Stützfunktion nicht mehr erfüllen, müssen sie operativ entfernt und durch Implantate ersetzt werden. Die dafür geeigneten Implantate müssen die mechanische Aufgabe des Stützens der benachbarten Wirbelkörper übernehmen. Dazu werden die Ersatzelemente zwischen die intakten Wirbelkörper eingebracht und an ihnen befestigt.

Beim Einsatz eines einzigen Wirbelkörpers reicht es aus, ein in Wirbelsäulenachse gerades Implantat einzusetzen. Müssen allerdings mehrere Wirbelkörper gleichzeitig ersetzt werden, z. B. bei ausgedehnten Tumorentfernungen ist es erforderlich, die Krümmung der Wirbelsäule im Stützimplantat zu berücksichtigen. Je nach Lokalisation (Halswirbelsäule, Brustwirbelsäule, Lendenwirbelsäule) muß sich die Kyphose bzw. Lordose im Implantat fortsetzen.

Aus der GB-PS 1 243 353 ist eine Ausführung bekannt, bei der das Implantat aus Elementen im Baukastenprinzip besteht, die miteinander verschraubt und über eine gekrümmte, mit Langlöchern versehene Schiene fixiert werden derart, daß das zusammengesetzte Implantat der gewünschten Wirbelsäulenkrümmung entspricht. Das Zusammensetzen der einzelnen Elemente zu einem Implantat benötigt jedoch eine zeitraubende Handhabung, die zudem während der Operation durchgeführt werden muß.

Ein hiergegen einfaches Implantat ist aus der DE 36 37 314 bekannt, das aus einem zylinderförmig aufgerollten Gitterelement besteht. Dieses bekannte Implantat muß jedoch insbesondere bei Ersatz von mehreren Wirbelkörpern mit einem Knochenzement gefüllt werden, um eine Verbiegung des Implantats zu vermeiden. Einwachsender Knochen würde die Stützfunktion nicht übernehmen können, da dessen Stützwirkung nicht ausreicht.

Eine andere Ausführung ist aus der US-PS 4,932,975 bekannt, die einen starren, zentralen Hauptträger enthält, an dessen Enden über elastiche Zwischenteile je ein Stützelement befestigt ist. Das elastische Zwischenteil erlaubt eine Schrägstellung der Stützelemente gegenüber der Hauptachse, so daß damit deren Auflageebenen sich an die Winkellage der jeweils aufliegenden Wirbelkörper anpassen können. Die Rückstellkräfte der elastischen Zwischenteile wirken jedoch kontinuierlich als eine unphysiologische Belastung auf die angrenzenden Wirbelkörper.

Den vorstehenden Nachteil vermeidet ein aus dem DE-Gbm 91 07 494 bekanntes Implantat, das aus einer Schraubenstange mit gegenläufigem Gewinde als zentralen Hauptträger besteht, wobei die Enden der Schraubenstange jeweils in eine Gelenkkugel einschraubbar sind, die je in einem Stützelement lagern. Die Neigung der Stützelemente wird hier ohne Federkräfte erreicht. Das Schraubsystem erlaubt jedoch keine Anpassung an Krümmungen der Wirbelsäule.

Der Erfindung liegt die Aufgabe zugrunde, ein Implantat der eingangs genannten Art zu entwickeln, das mit möglichst einfacher Handhabung implantiert und ohne unerwünschte Kräfteeinwirkungen an die anatomatischen Bedingungen der Wirbelsäule anpaßbar als Ersatz von einem oder mehreren Wirbelkörpern verwendet werden kann.

Die Aufgabe wird erfindungsgemäß durch die Merkmale des Anspruchs 1 gelöst.

Das erfindungsgemäße Implantat besteht aus drei Bauelementen, die nach Einstellung der Winkellage der Auflageebenen mindestens eines Stützelementes des Implantats starr miteinander verbunden werden. Die Ausrichtung der Stützelemente kann vor der Implantierung oder im implantierten Zustand erfolgen. Ein derartiges Implantat ist geeignet, auch als Ersatz von mehreren Wirbeln eine gute Anpassung an die Anatomie zu gewährleisten und als starres Bauteil ohne unerwünschte Belastungen die notwendige Stütze zu bieten.

Bei der Verwendung des erfindungsgemäßen Implantats als Ersatz für mehrere nebeneinanderliegende Wirbelkörper ist es von Vorteil, wenn der Hauptträger gekrümmt ausgebildet ist, insbesondere die Krümmung der Wirbelsäule an der Operationsstelle annimmt.

Zur Realisierung des erfindungsgemäßen Implantats sind verschiedene Ausführungsvarianten möglich.

Eine fertigungstechnisch sehr einfache Ausgestaltung besteht in einem annähernd zylindrischen Hauptträger, der vorzugsweise über die Länge gekrümmt ist und mit dessen Stirnseiten die Stützelemente in der gewünschten Neigung verbindbar sind.

Der Querschnitt des annähernd zylinderförmigen Hauptträgers ist vorzugsweise nierenförmig, wodurch eine erhöhte Sicherheit gegen Verletzungen des Rückenmarks erreicht wird.

Der Hauptträger kann als Hohlkörper ausgebildet und aus faserverstärktem Verbundwerkstoff hergestellt sein. Durch Anwendung der Wickeltechnik und/oder Flechttechnik kann eine selbsttragende Konstruktion hergestellt werden, die kein die mechanische Stabilität erhöhendes Material, wie Knochenzement, benötigt. Die Anwendung einer Schichttechnik, z. B. die Verbindung eines Rohres mit einem Netzwerk oder dergleichen, ist ebenfalls möglich.

Der Hauptträger kann bei Bedarf auch spreizbar ausgeführt werden, wobei bekannte Schraub-Spreizmechanismen zur Anwendung kommen können. Gemäß einer Ausgestaltung der Erfindung ist der Hauptträger teleskopartig ausgebildet und mit einem Ventilanschluß versehen, über den ein Druckmedium in den Hohlraum zum Auseinanderdrücken der Teleskoprohre eingebracht werden kann. Bei Verwendung eines aushärtbaren Druckmittels, z. B. Knochenzement, Harz, können die freibeweglich bzw. gelenkig beweglichen Stützelemente zwischen den Wirbelkörpern während des Spreizvorganges direkt durch die angrenzenden Wirbelkörper automatisch gerichtet und durch Aushärten des Druckmittels in der Lage fixiert werden. Damit entfallen vorhergehende Messungen der Winkellagen der angrenzenden Wirbelkörper, insbesondere wenn mehr als ein Wirbelkörper durch das Implantat überbrückt werden soll.

Das erfindungsgemäße Implantat erlaubt ferner eine Vielzahl von Verankerungs- und Ausgestaltungsmöglichkeiten für die Anbindung der Stützelemente am Hauptträger.

Gemäß einer einfachen Ausgestaltung der Erfindung sind die Stirnenden des Hauptträgers konkav oder konvex mit halbkugeliger Kontur ausgebildet, während die Stützelemente die Gegenform erhalten. Auf diese Weise kann durch Gleitverschiebung auf dem Dom des Hauptträgers die Orientierung der Auflageebene eines Stützelementes stufenlos verändert und dadurch eine Orientierung eingestellt werden, bei der die Auflageebenen der beiden Stützelemente den Winkel einnehmen, den die dem Implantat anschließenden Wirbelkörper des Patienten bilden.

Ferner sind Kugelgelenke möglich, bei denen den Stützelementen je eine Kugel zugeordnet ist, die in einer Kalotte am Hauptträger in allen Richtungen schwenkbar gelagert ist. Mittels Schraubverbindungen, auch unter Anwendung von Schraubkappen, können verschiedene Bewegungs- und Fixierungsmechanismen kombiniert werden. So kann beispielsweise eine ausgesuchte Position eines Kugelgelenkes mittels einer Schraubkappe am Hauptträger fixiert werden.

Gemäß einem weiteren erfindungsgemäßen Beispiel wird am Dom des Hauptträgers eine Schlitzöffnung vorgesehen, die zur Einstellung des richtigen Winkels eine Schwenkbewegung des Stützelementes samt einer mit dem Stützelement verbundenen Schraube zuläßt.

Gemäß einer weiteren Ausgestaltung der Erfindung ist eine Klebverbindung vorgesehen, die durch direktes Aufkleben des Stützelementes auf den Dom erfolgt.

Vorzugsweise wird für die Klebverbindung sowohl im Hauptträger als auch im Stützelement ein Raum zur Aufnahme von Klebmaterial vorgesehen. Bei einem vollen Hauptträger wird dazu im Dom eine Bohrung vorgesehen, während im Stützelement eine durchgehende Öffnung eingebracht ist, durch die Klebmaterial, z. B. Knochenzement nach der Positionierung des Stützelementes eingebracht wird. Eine stabile formschlüssige Verbindung wird damit erreicht.

Ein hohlausgebildeter Hauptträger hat dazu an den Stirnenden je eine Bohrung und im Hohlkörper eine innere Abschlußplatte zur Reduzierung des Füllraumes. Die Abschlußplatte kann aber auch weggelassen und der gesamte Hohlraum voll mit Knochenmaterial (keine Stützfunktion) gefüllt werden, so daß nach dem Knocheneinwachsen eine Säule entsteht. Die Fixierung des Stützelementes durch ein in den Hauptträger einzuführenden Klebemittels wird insbesondere durch einen Verankerungsstab sichergestellt, der mit dem Stützelement fest verbunden ist und vom Klebemittel umgeben wird.

Bei einem mit einem Druckmedium spreizbaren Implantat kann das Stützelement ebenfalls einen in den Innenraum des Hauptträgers hineinragenden Verankerungsstab haben, der in das Durckmedium, insbesondere ein aushärtbares Mittel, eintaucht und dadurch fixiert wird. Es sind aber auch andere Mechanismen denkbar, wie Druckplatten, die aufgrund des Druckes eines eingeführten Mediums (auch ein nicht aushärtbares Fluid) eine reibschlüssige Verbindung zwischen dem Stützelement und dem Hauptträger herstellen.

Mit dem erfindungsgemäßen Implantat in seinen vielfältigen Ausführungen ist eine optimale Anpassung an die anatomischen Gegebenheiten ohne aufwendige Arretierungsvorrichtungen möglich.

Das Implantat eignet sich ferner zur Aufnahme von mechanischen Spreizsystemen. Bei einem Hauptträger mit rundem Querschnitt kann eine doppelseitige Hohlschraube mit inneren oder äußeren gegenläufigen Gewinden verwendet werden, in bzw. auf die die beiden Teile eines quergetrennten Hauptträgers ein- bzw. aufgeschraubt werden. Spreizelemente dieser Art sind bekannt (z.B. US 4,553,273, US 4,401,112).

Es ist auch denkbar, daß der Spreizvorgang nicht am Hauptträger, sondern an einem der Stützelemente erfolgt. Eine einfache Konstruktion besteht in einer an einem Teil des Stützelementes rotationsbeweglich fixierten Mutter mit Innengewinde am freien Ende, in die das zweite Teil des Stützelementes einschraubbar ist.

In der Zeichnung sind Ausführungsbeispiele der Erfindung schematisch dargestellt. Es zeigen:
- Fig. 1: ein erstes Ausführungsbeispiel,
- Fig. 2: einen Querschnitt aus Fig. 1 und
- Fig. 3 bis 9: je ein weiteres Ausführungsbeispiel.

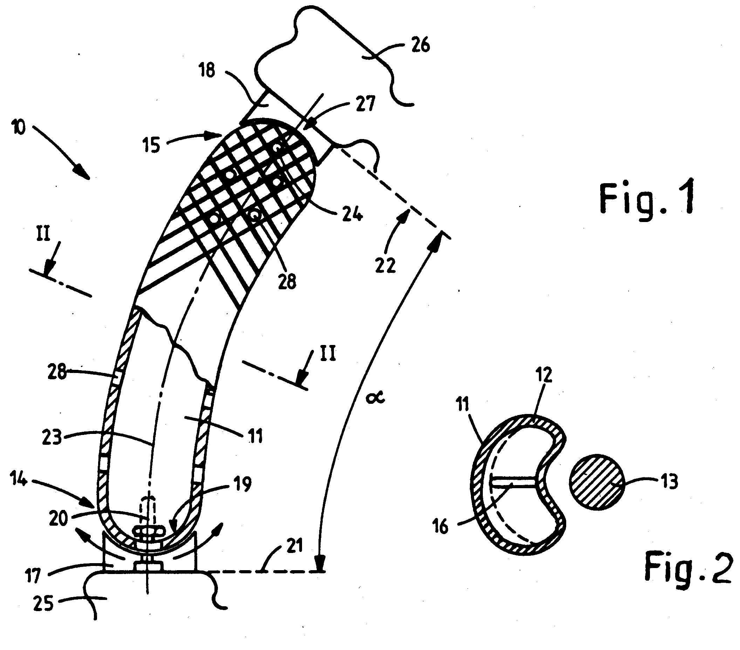

Fig. 1 zeigt ein Wirbelkörperimplantat 10, bestehend aus einem Hauptträger 11 aus faserverstärktem Kunststoff, beispielsweise aus C-Fasern, der zur Überbrückung von zwei oder mehreren aus einem Patienten herausoperierten Wirbelkörpern eine längliche Form aufweist und entsprechend der Krümmung einer Wirbelsäule leicht gebogen ist.

Wie in Fig. 2 gezeigt ist, ist der Querschnitt 12 des Hauptträgers 11 nierenförmig ausgebildet, um eine ausreichende Distanz zum Rückenmark 13 zu gewährleisten.

Die Stirnenden bzw. Dome 14 und 15 des Hauptträgers 11 sind halbkugelförmig ausgebildet und enthalten je ein Langloch 16 (Fig. 2) zur Fixierung von jeweils einem Stützelement 17 bzw. 18, die hauptträgerseitig halbkugelige Ausnehmungen 19 und 24 aufweisen. Mit einer Schraube 20 wird das Stützelement 17 mit dem Hauptträger 11 fest verbunden. Die Stützelemente 17, 18 sind in Pfeilrichtung, d. h. parallel zum Langloch 16 um das Domzentrum schwenkbar, wodurch sich die Orientierung der Auflageebenen 21 bzw. 22 der Stützelemente 17 bzw. 18 an die Winkellage α der anschließenden Wirbelkörper 25, 26 des Patienten anpassen läßt.

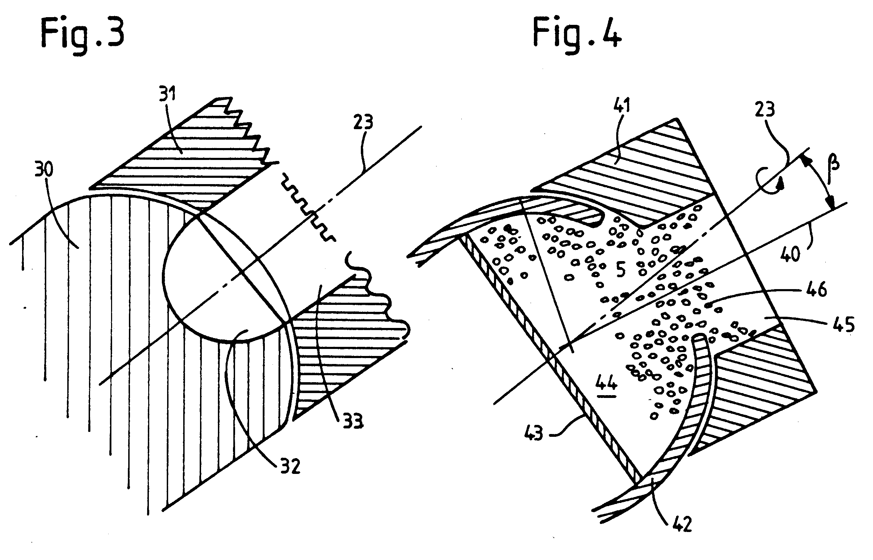

Der Hauptträger 30 kann ein Hohlkörper gemäß Fig. 1 oder als volles Bauteil ausgebildet sein, wie er beispielsweise in Fig. 3 gezeigt ist. Für den Aufbau des Hauptträgers sind homogene sowie auch schichtartige Strukturen möglich.

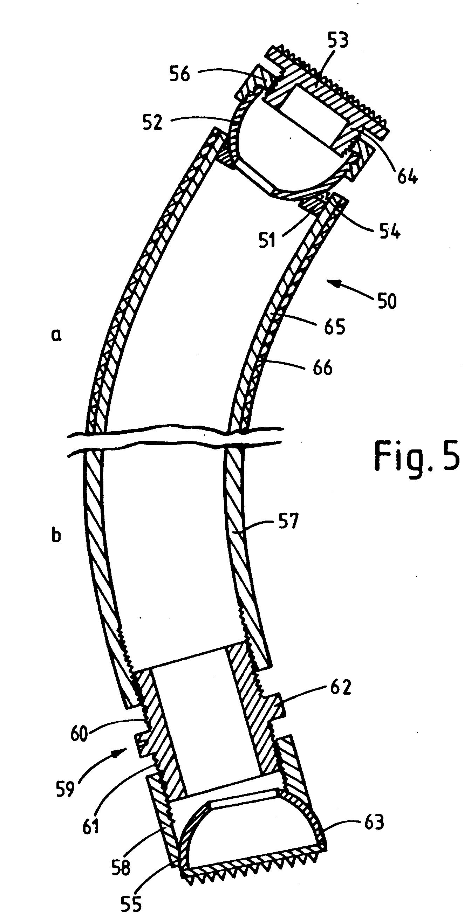

Fig. 3, 4 und 5a zeigen Hauptträger 30, 42, 57 mit homogener Struktur. Der Hauptträger 50 gemäß Fig. 5a ist dagegen aus zwei Schichten aufgebaut, z. B. aus einem Rohr 65 und einer netzartigen Außenschicht 66.

Fig. 3 zeigt eine Variante, bei der das Stützelement 31 direkt auf den Dom des Hauptträgers 30 aufgeklebt wird. Zur Verstärkung der Formschlüssigkeit können Hauptträger 30 und Stützelement 31 mit kommunizierenden Hohlräumen 32 bzw. 33 versehen werden, die mit einem Klebmaterial, z. B. Knochenzement oder -material gefüllt werden.

In der Regel wird die Achse 40 des Stützelementes 41 um einen Winkel β von der Hauptträgerachse 23 verdreht sein, wie es in Fig. 4 in Verbindung mit einem als Hohlkörper ausgebildeten Hauptträger 42 dargestellt ist. Um die Klebmaterialmenge zu reduzieren, ist in den Dombereichen des Hauptträgers 42 je eine interne Abschlußplatte 43 vorgesehen. Nach Plazierung des Stützelementes 41 um den gewählten Winkel β wird der im Hauptträgerdom verbleibende Hohlraum 44 und der Durchbruch 45 des Stützelementes 41 mit dem Klebmaterial 46 gefüllt, das vor oder nach der Implantierung ausgehärtet wird.

Der Hohlkörper 42 kann aber auch vollständig mit Knochenmaterial gefüllt werden, so daß eine Knochensäule entsteht, nachdem der Knochen eingewachsen ist. Eine Stützfunktion erfüllt die Knochensäule nicht.

Es ist ferner eine Umkehrung der Kugelflächen am Hauptträgerdom und den Stützelementen möglich, bei dem die Hauptträgerdome konkav und die Stützelemente hauptträgerseitig konvex ausgebildet werden. Ausführungsbeispiele in Verbindung mit einem rohrförmigen Hauptträger sind in Fig. 5 gezeigt.

Gemäß Fig. 5a ist im Hauptträgerrohr 50 ein Stützring 51 vorgesehen, dessen Innenwand zur Aufnahme des halbkugelförmigen Teils 52 des Stützelementes 52, 53 kugelschichtförmig ausgebildet ist, und mit Klebmaterial bestrichen wird, um das Stützelement 52, 53 nach dem Einstellen dessen Winkelstellung zu fixieren. Ein vorgesehener Ringspalt 54 am Ende des Hauptträgers wird zur Verstärkung der Klebverbindung mit Klebmaterial gefüllt. Anstelle des Stützringes 51 kann ein kugelschichtförmig geformter Stirnrand 55 des Hauptträgerrohres 58 (Fig. 5b) als Abstützung des Stützelementes 63 dienen.

Bei einem massiven Hauptträger wird das Stützelement in eine halbkugelförmige Ausnehmung an der Stirnseite des Hauptträgers geklebt.

Günstig ist ferner, wenn das Implantat mit einem Spreizmechanismus ausgestattet ist. Beispiele zu dessen Realisierung sind in Fig. 5 gezeigt.

In Fig. 5a ist eine Ausführung gezeigt, bei der das Stützelement in einem halbkugelförmigen Teil 52 und einer Auflageplatte 53 aufgeteilt ist, die mittels einer Mutter 56 miteinander verbunden sind. Die Mutter 56 ist axial formschlüssig und in Umfangsrichtung drehbeweglich mit dem halbkugelförmigen Teil 52 verbunden. Am freien Ende enthält die Mutter 56 ein Innengewinde zur Aufnahme eines mit Außengewinde versehenen Ringstutzens 64 der Auflageplatte 53. Durch Drehen der Mutter 56 ist die Auflageplatte 53 axial verschiebbar. Dieser Spreizmechanismus ist nur an einem Stützelement anzubringen.

Gemäß Fig. 5b wird der Spreizvorgang am Hauptträger 57 durchgeführt. Dazu ist der Hauptträger in zwei Teile 57 und 58 geteilt und mittels einer zweiseitigen Hohlschraube 59 miteinander verbunden. Die Schraube 59 hat zwei gegenläufige Gewinde 60 und 61 und einen zentralen Ring 62 mit Mehrkantumfang oder Bohrungen, zum Ansetzen eines Werkzeugs, mit dem die Schraube 59 im implantierten Zustand gedreht werden kann, um das Implantat zu spreizen. Die Stützelemente 63 sind in diesem Fall einstückig und starr mit dem Hauptträger 58 verbunden.

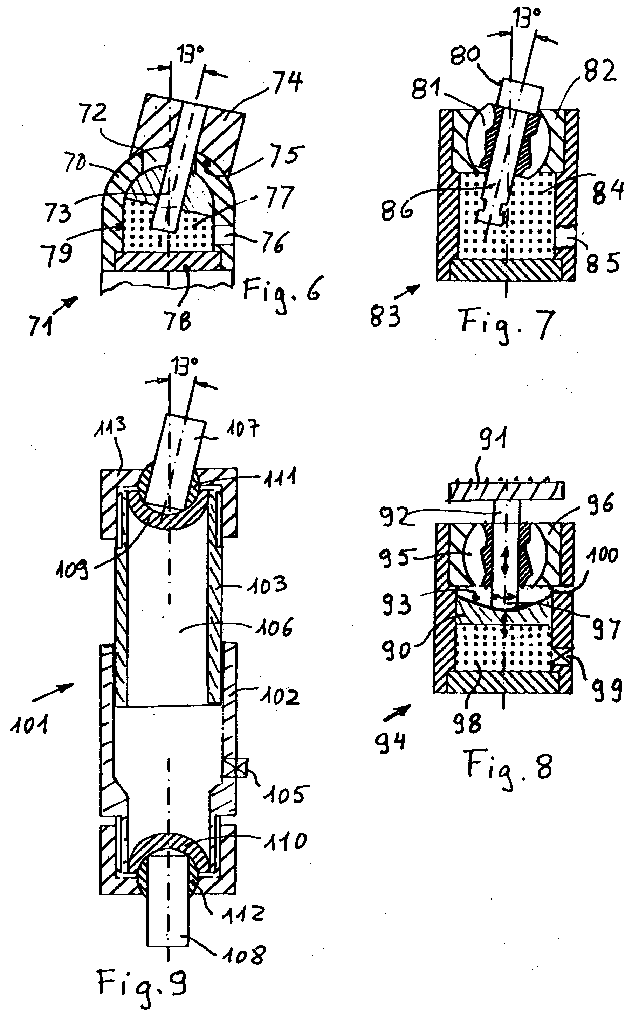

Fig. 6 zeigt einen konvex geformten Dom 70 seines Hauptträgers 71, der eine zentrale Bohrung 72 aufweist, durch die ein Verankerungsstab 73 des Stützelementes 74 in einen Hohlraum 75 des Hauptträgers 71 hineinragt. Das Stützelement 74 kann aufgrund seiner konvexen Verbindungsfläche 75 und der überdimensionierten Bohrung 72 in allen Richtungen um etwa 26° geschwenkt werden. Über eine Öffnung 76 wird ein aushärtbares Mittel 77 in den Hohlraum 75 des Hauptträgers 71 eingebracht, das nach dem Aushärten den Verankerungsstab in der gewünschten Position fixiert. Um die notwendige Menge des Fixierungsmittels 77 zu beschränken, ist der Hohlraum 75 des Hauptträgers 71 mittels einer Trennwand 78 oder Abschlußplatte in der Nähe des Domes 70 abgetrennt.

Fig. 7 zeigt ein Ausführungsbeispiel, bei dem das Stützelement 80 über ein Kugelgelenk 81, 82 dreh- und schwenkbeweglich mit dem Hauptträger 83 verbunden ist. Auch in diesem Fall ist die Verankerung mit einem Klebmittel 84 vorgesehen, das durch eine Öffnung 85 in der Wandung des Hauptträgers 83 zur Fixierung des Verankerungsstabes 86 in dessen Umgebung einführbar ist. Kurze Implantate werden in der Regel nur mit einem beweglichen bzw. einstellbaren Stützelemment ausgestattet, wobei das zweite Stützelement starr mit dem Hauptträger verbunden oder durch das andere Ende des Hauptträgers direkt gebildet ist.

Fig. 8 und 9 zeigen Ausführungsbeispiele, bei denen das Stützelement bzw. der Hauptträger als Spreizvorrichtungen ausgebildet sind.

Gemäß Fig. 8 dient ein mit einem Kolben 90 verbundenes Stützelement 91 als Spreizelement. Das plattenförmig ausgebildete Stützelement 91 ist über den Verankerungsstab 92 in einer konkaven Fläche 93 des Kolbens 90 abgestützt und im Hauptträger gelenkig gelagert. Hierzu ist der Verankerungsstab 92 mit einer Gelenkkugel 95 fest verbunden, die in einer mit dem Hauptträger 94 verbundenen Ringkalotte 96 dreh- und schwenkbeweglich gelagert ist, so daß das Stützelement 91 sich im implantierten Zustand an den angrenzenden Wirbeln ausrichten kann. Dabei gleitet das freie Ende 97 des Verankerungsstabes 92 auf der konkaven Fläche 93 des Kolbens 90. Durch Einführung eines Druckmittels 98 durch ein Ventil 99 des Hauptträgers 94 wird der Kolben 90 in Richtung des Hauptträgerdomes gedrückt, so daß die Kraft über den Verankerungsstab 92 auf das Stützelement 91 übertragen wird. Über eine weitere Öffnung 100 kann ein aushärtbares Mittel auf die konkave Seite 93 des Kolbens 90 zur Fixierung des Verankerungsstabes 92 eingebracht werden. Bei der Ausführung gemäß Fig. 8 reicht es, wenn nur ein Stützelement 91 axial verschiebbar ausgebildet ist.

Gemäß Fig. 9 ist der Hauptträger 101 als Spreizelement aus zwei teleskopartig ineinanderschiebbaren Rohren 102 ausgebildet. Überein Ventil 105 wird ein Druckmittel, das ein Gas, eine Flüssigkeit oder ein aushärtbares Mittel, wie Knochenzement sein kann, unter Druck eingeführt und damit das Implantat über sein Stützelement gegen die angrenzenden Wirbelkörper gespreizt.

Bei dieser Ausführung werden beide Enden des Implantats in Bewegung gesetzt werden. Der im Innenraum 106 des Hauptträgers 101 herrschende Druck kann gleichzeitig zur reibschlüssigen Verankerung der Stützelemente 107, 108 genützt werden, indem beispielsweise die Kalotten 109, 110 für die Kugelgelenke 111, 112 mit speziell ausgebildeten Reibflächen gegen die Gelenkkugeln 111, 112 gedrückt werden.

Es wird nicht immer erforderlich sein, beide Stützelemente schwenkbeweglich auszuführen. Jedoch wird bei dem Ersatz von größeren Wirbelkörpern, in der Umgebung der Lendenwirbelsäule sowie beim Ersatz von mehreren Wirbelkörpern eine beiderseitige Schwenkbeweglichkeit der Stützelemente der Anatomie der Wirbelsäule gerechter.

## Patentansprüche

1. Implantat für den Ersatz von Wirbelkörpen, bestehend aus einem zentralen Hauptträger, an dessen beiden Enden (14 bzw. 15) je ein Stützelement (17, 18 bzw. 31, 41, 52, 63) vorgesehen ist, wobei mindestens ein Stützelement eine Auflageebene mit einstellbarer Winkellage der Auflageebene für einen anschließenden Wirbelkörper hat, dadurch gekennzeichnet, daß das oder die Stützelemente (17, 18, 31, 41, 52, 63) nach Einstellung der Winkellage (α, β) der Auflageebene (27) mit dem Hauptträger starr verbindbar sind.

2. Implantat nach Anspruch 1, dadurch gekennzeichnet, daß der Hauptträger (11, 30, 42, 50, 57) der Krümmung des menschlichen Rückgrads annähernd folgend ausgebildet ist, daß der Hauptträger(11, 42, 50, 57) als Hohlkörper, insbesondere in der Form eines Zylinders ausgebildet ist, dessen Wandung (11, 42, 57 bzw. 65 und 66) ein- oder mehrschichtig ausgebildet ist und ferner, daß die Wandung gegebenenfalls Durchbrüche (28) aufweist oder gitterartig ausgebildet ist.

3. Implantat nach Anspruch 1 oder 2, dadurch gekennzeichnet, daß die Stützelemente über Kugelgelenke mit dem Hauptträger verbunden sind.

4. Implantat nach einem der vorhergehenden Ansprüche, dadurch gekennzeichnet, daß die Stützelemente (17, 18) an den Hauptträger (11) anschraubbar (20) sind.

5. Implantat nach einem der Ansprüche 1 bis 3, dadurch gekennzeichnet, daß die Stützelemente (31, 41, 52, 63) mittels einer Kleb-, Reib- oder Formschlußverbindung am Hauptträger (30, 42, 50 bzw. 57, 58) befestigbar sind und daß gegebenenfalls für die Verbindungen Hohlräume (32, 33 bzw. 44, 45) vorgesehen sind, die Füll- oder Klebmaterial (43) aufnehmen.

6. Implantat nach einem der vorhergehenden Ansprüche, dadurch gekennzeichnet, daß der Hauptträger (11, 30, 42, 50, 57) und/oder die Stützelemente (17, 18, 31, 41, 52, 53, 63) aus Faserverbundwerkstoff, insbesondere C-Faserverbund, bestehen.

7. Implantat nach einem der vorhergehenden Ansprüche, dadurch gekennzeichnet, daß der Hauptträger (57, 58) oder mindestens ein Stützelement 52, 53) mit einem Spreizmechanismus (59 bzw. 56) ausgestattet oder als Spreizelement (Fig. 8, 9) ausgebildet ist.

8. Implantat nach Anspruch 7, dadurch gekennzeichnet, daß der Hauptträger (101) teleskopartig ausgebildet ist und einen Anschlag (105) zur Einführung eines Druckmittels aufweist.

9. Implantat nach Anspruch 7, dadurch gekennzeichnet, daß zumindest ein Stützelement (91) mittels eines Kolbens (90) axial verschiebbar ausgebildet ist.
